# FASCICULE DE BREVET EUROPEEN

(11) **EP 4 276 459 B1**
(45) Date de publication et mention de la délivrance du brevet: **03.07.2024**
(21) Numéro de dépôt: 23171287.8
(22) Date de dépôt: 03.05.2023
(51) Int. Cl.: G01N 33/08, A01K 43/00

(54) **PROCÉDÉ ET SYSTÈME DE DÉTECTION DE LA PRÉSENCE D'UN ORGANE D'UN EMBRYON DANS UN OEUF**
VERFAHREN UND SYSTEM ZUR ERKENNUNG DER ANWESENHEIT EINES ORGANS EINES EMBRYOS IN EINEM EI
METHOD AND SYSTEM FOR DETECTING PRESENCE OF EMBRYO ORGAN IN EGG

(30) Priorité: 12.05.2022 FR 2204490
(43) Date de publication de la demande: 15.11.2023
(73) Titulaire: Commissariat à l'énergie atomique et aux énergies alternatives, 75015 Paris (FR)
(72) Inventeur: REDON, Olivier, 38054 Grenoble cedex 09 (FR); CAILLY, Alexis, 38054 Grenoble cedex 09 (FR); MAKKI, Ihab, 38054 Grenoble cedex 09 (FR)
(74) Mandataire: INNOV-GROUP

(56) Documents cités:
- WO-A1-2015/073939
- WO-A1-2016/005539
- WO-A1-2019/170887
- CN-A- 104 316 473
- CORION MATTHIAS ET AL: "In ovo sexing of eggs from brown breeds with a gender-specific color using visible-near-infrared spectroscopy: effect of incubation day and measurement configuration", POULTRY SCIENCE, vol. 101, no. 5, 1 mai 2022 (2022-05-01), page 101782, XP055962758, Oxford ISSN: 0032-5791, DOI: 10.1016/j.psj.2022.101782

## Description

### Domaine technique de l'invention

La présente invention se rapporte à un procédé et à un système de détection de la présence d'un organe d'un embryon dans un oeuf, mis en oeuvre de manière non invasive.

Le procédé et le système sont notamment parfaitement adaptés pour déterminer in fine le sexe d'un oeuf de canard, de manière non invasive.

### Etat de la technique

La recherche d'une technique de sexage précoce et non invasive des oeufs de volailles est un sujet qui intéresse l'industrie volaillère depuis plusieurs années.

Des techniques invasives ont été proposées dans l'état de la technique, notamment dans les demandes de brevets WO98/14781, DE102007013102, WO2010/103111, US2011/0144473A1, WO2017/174337

Des techniques optiques non invasives ont également été proposées mais elles concernent principalement la détection de la fertilité des oeufs et non le sexage. Sur ce sujet, on peut notamment citer le brevet US 9,435,732 et le brevet US 6,029,080.

Dans la filière palmipède pour la production de foie gras, le canard mulard a été spécifiquement sélectionné car les mâles transmettent un gène albinos uniquement aux femelles, ce qui permet de les sexer après éclosion des canetons par la couleur de l'oeil : Les mâles ont en effet des yeux noirs car ils contiennent de la mélanine et les femelles albinos ont les yeux rouges. Cette particularité permet d'envisager un sexage in ovo autour du 9^{eme} jour en effectuant un mirage des oeufs. En agitant l'œuf il est alors possible d'amener l'œil au contact de la coquille ce qui permet de le distinguer.

Dans l'état de l'art, le mirage pour le sexage utilise une diode électroluminescente visible qui éclaire l'œuf par le haut, l'œuf étant positionné à 45°, et une caméra positionnée à 90° de l'axe de symétrie de l'œuf est destinée à capter l'image de diffusion de la lumière. Cette configuration n'est pas idéale car l'homogénéité de l'éclairage dans l'œuf est mauvaise. En effet, comme la lumière est diffusée dans toutes les directions, plus le chemin de la lumière dans l'œuf est long, plus l'absorption de la lumière est importante. On obtient dès lors un gradient d'intensité du haut vers le bas qui est d'autant plus renforcé que la lumière doit traverser le jaune, celui-ci étant plus absorbant que le blanc. L'oeil de l'embryon qui surnage à la surface du jaune apparait donc à la limite de la zone sombre, ce qui gêne sa détection. La forte absorption du jaune à ce stade de l'incubation vient du développement du système cardio-vasculaire qui contient de l'hémoglobine, présentant une très forte d'absorption dans la gamme de longueurs d'ondes de l'éclairage de la diode électroluminescente entre 400 et 620nm.

Grâce à une combinaison de mirage et d'agitation autour du 9^{eme} jour, on parvient à observer l'œil chez les mâles mais cette technique souffre de plusieurs inconvénients :
- Le mirage avec une diode électroluminescente et une caméra monochrome ne permet pas d'obtenir un bon contraste de l'œil par rapport au système cardio-vasculaire, ce qui est source d'erreur de prédiction.
- Le processus d'agitation est souvent assez violent, ce qui peut être dommageable pour l'embryon et peut venir affecter le taux d'éclosabilité.
- Plus l'incubation avance, plus l'embryon a tendance à s'enfoncer dans le jaune, ce qui complique le processus d'agitation. Le mouvement généralement utilisé pour ramener l'œil à la surface est un mouvement angulaire autour de l'axe de l'œuf, ce qui complexifie son déploiement industriel.

La publication référencée "CORION MATTHIAS et AL : In ovo sexing of eggs from brown breeds with a gender-specific color using visible-near-infrared spectroscopy : effect of incubation day and measurement configuration", POULTRY SCIENCE, col. 101, n°5; 1 mai 2022 page 101782, XP055962758, Oxford*,* décrit également une technique de sexage des oeufs de poule par analyse spectroscopique.

Ce document antérieur décrit une technique de sexage de la poule par l'eumélanine contenue grande quantité dans les plumes des femelles. La solution est basée sur une analyse de données spectrales. Dans cette approche, on s'applique à rechercher les signatures spectrales les plus marquantes pour la prédiction du sexe. La publication CN 104 316 473 A décrit une technique de sexage par imagerie hyperspectrale.

Le but de l'invention est de proposer un procédé de détection de la présence d'un organe d'un embryon au sein d'un oeuf, qui soit non-invasif, facile à mettre en oeuvre, et qui soit parfaitement fiable dans la détection.

### Exposé de l'invention

Ce but est atteint par un procédé non-invasif de détection de la présence d'un organe d'un embryon au sein d'un oeuf, le procédé comportant :
- Une étape d'émission à travers l'œuf, par une source d'émission, d'un signal lumineux,
- Une étape de filtrage du flux lumineux dans une bande spectrale étroite autour d'une longueur d'onde de 700nm,
- Une étape d'imagerie de l'œuf traversé par le flux lumineux,
- Une étape d'analyse de l'image de l'œuf générée lors de l'étape d'imagerie,
- La présence dudit organe étant révélée par contraste d'absorption entre les composantes constitutives de l'œuf contenant du sang et ledit organe de l'embryon contenant de la mélanine.

Le procédé s'appuie sur une analyse de l'image de l'œuf, en vue de détecter au sein de l'œuf le contraste entre ledit organe de l'embryon contenant de la mélanine et les autres organes contenus dans l'œuf.

Le procédé permet plus particulièrement un effacement des composantes constitutives d'un oeuf contenant du sang, pour mettre en valeur l'organe de l'embryon contenant la mélanine.

Comme l'absorption de la mélanine est quasiment constante sur toute la gamme spectrale 400nm-1100nm (gamme VNIR), il n'y a pas de longueur d'onde réellement préférentielle pour la détecter. En revanche, il est possible de fortement réduire la visibilité des organes présents dans l'œuf qui contiennent du sang et ainsi de révéler par contraste la présence de la mélanine.

Selon une réalisation particulière, la bande spectrale étroite est comprise entre 650nm et 750nm.

Selon une autre réalisation particulière, la bande spectrale étroite est comprise entre 675nm et 725nm.

Selon une autre réalisation particulière, l'étape de filtrage est mise en oeuvre sur le flux lumineux émis par la source d'émission, avant transmission dans l'œuf.

Selon une autre réalisation particulière, l'étape de filtrage est mise en oeuvre sur le flux lumineux émis par la source d'émission, après transmission à travers l'œuf et avant l'étape d'imagerie.

Selon une autre réalisation particulière, le procédé comporte une étape mise en mouvement de l'œuf, contenant des accélérations de l'œuf devant la source d'émission afin de ramener ledit organe contenant de la mélanine au plus près de la coquille. Selon une autre réalisation particulière, le procédé consiste à déterminer le sexe d'un oeuf de canard mulard à partir de la détection de présence de l'œil dudit canard.

L'invention concerne également un système non-invasif de détection de la présence d'un organe d'un embryon au sein d'un oeuf, le système comportant :
- Un élément de maintien agencé pour maintenir l'œuf dans une position,
- Une source d'émission d'un signal lumineux à travers l'œuf maintenu en position,
- Un dispositif de réduction de la bande spectrale du signal lumineux émis par la source dans une bande spectrale étroite autour d'une longueur d'onde de 700nm,
- Un dispositif d'imagerie configuré et agencé pour recevoir un flux lumineux après transmission à travers l'œuf,
- Des moyens d'analyse de l'image de l'œuf obtenue à l'aide du dispositif d'imagerie, configurés pour détecter au sein de l'œuf un contraste d'absorption entre les composantes constitutives de l'œuf contenant du sang et ledit organe de l'embryon contenant de la mélanine.

Selon une réalisation particulière, le dispositif de réduction de la bande spectrale est un filtre centré sur une longueur d'ondes de 700nm et de largeur de bande de 100nm. Selon une autre réalisation particulière, le dispositif de réduction de la bande spectrale est situé entre la source d'émission et l'élément de maintien en position de l'œuf. Selon une autre réalisation particulière, le dispositif de réduction de la bande spectrale est situé entre l'élément de maintien en position de l'œuf et le dispositif d'imagerie. Selon une autre réalisation particulière, le système comporte des moyens de mise en mouvement de l'élément de maintien, configurés pour créer des accélérations.

Selon une réalisation particulière, l'élément de maintien est configuré pour maintenir l'œuf de sorte que le plus grand axe de l'œuf soit parallèle à un plan horizontal.

Selon une particularité, la source d'émission est positionnée pour émettre le signal lumineux suivant l'axe d'émission perpendiculaire audit plan horizontal.

Selon une autre particularité, la source d'émission est positionnée de manière à pouvoir éclairer l'œuf par le bas.

Selon une autre réalisation particulière, caractérisé en ce que l'élément de maintien est adapté pour incliner l'œuf de sorte que son plus grand axe soit orienté à 45° par rapport à une direction verticale.

Selon une particularité, la source d'émission est positionnée pour émettre le signal lumineux suivant l'axe d'émission incliné à 45° par rapport à la direction verticale. Selon une autre réalisation particulière, l'élément de maintien est adapté pour maintenir l'œuf de sorte que le plus grand axe de l'œuf soit orienté suivant une direction verticale. Selon une particularité, la source d'émission est positionnée pour émettre le signal lumineux suivant l'axe d'émission selon la direction verticale.

Selon une autre réalisation particulière du système, le capteur est positionné dans l'axe d'émission de la source d'émission.

Selon une autre réalisation particulière du système, le capteur est positionné pour recevoir le flux lumineux à 90° par rapport à l'axe d'émission.

Selon une particularité, le système comporte des moyens de mise en mouvement relatif dudit élément de maintien par rapport audit capteur.

Selon une autre particularité, les moyens de mise en mouvement comportent des moyens de mise en vibration de l'élément de maintien.

Selon une autre particularité, le capteur est une caméra monochrome configurée pour capter des images de l'œuf éclairé par ledit signal lumineux.

Selon une autre particularité, la source d'émission comporte une source lumineuse composée d'une ou plusieurs diodes électroluminescentes.

Selon une autre particularité, la source d'émission comporte au moins un filtre optique appliqué sur ladite source lumineuse ou sur le capteur et choisi pour obtenir ledit flux lumineux reçu par le capteur sur une plage de longueurs d'onde centrée autour de 700nm.

L'invention concerne également l'utilisation du système tel que défini ci-dessus, pour déterminer le sexe d'un oeuf de canard.

L'invention concerne également l'utilisation du système tel que défini ci-dessus, pour déterminer l'état de fécondation d'un oeuf dont l'embryon présente une concentration locale en mélanine.

### Brève description des figures

D'autres caractéristiques et avantages vont apparaître dans la description détaillée qui suit faite en regard des dessins annexés dans lesquels :
- La figure 1 représente de manière schématique un oeuf en incubation (entre J7 et J10) et son contenu et montre un mode de réalisation particulier et préféré du système de l'invention ;
- La figure 2A et la figure 2B représentent de manière schématique une première configuration de réalisation du système conforme à l'invention, appliqué sur un oeuf ;
- La figure 3A et la figure 3B représentent de manière schématique une autre configuration de réalisation du système conforme à l'invention, appliqué sur un oeuf ;
- La figure 4A et la figure 4B représentent de manière schématique une autre configuration de réalisation du système conforme à l'invention, appliqué sur un oeuf ;
- La figure 5 représente de manière schématique une autre configuration de réalisation du système conforme à l'invention, appliqué sur un oeuf ;
- La figure 6 représente de manière schématique une autre configuration de réalisation du système conforme à l'invention, appliqué sur un oeuf ;
- La figure 7 représente de manière schématique une autre configuration de réalisation du système conforme à l'invention, appliqué sur un oeuf ;
- La figure 8A montre un exemple de spectre d'émission de la source lumineuse employée et illustre la plage d'intérêt P centrée autour de 700nm et la figure 8B montre le spectre obtenu par un filtre passe-bande utilisé pour sélectionner la plage d'intérêt ;
- La figure 9 montre plusieurs photos capturées par la caméra du système de l'invention, à différents stades d'incubation de l'œuf et permet d'illustrer l'intérêt du procédé et du système de l'invention ;
- La figure 10 montre un exemple de réalisation d'une installation employée pour traiter plusieurs oeufs de manière simultanée ;

### Description détaillée d'au moins un mode de réalisation

Le système de l'invention est utilisé pour détecter la présence d'un organe d'un être vivant dans un oeuf O et permet, in fine, de déterminer par exemple le sexe (mâle ou femelle) de l'être vivant ou l'état de fécondation. Il est notamment parfaitement adapté pour le sexage d'un oeuf de canard de type mulard. Mais il peut également être utilisé pour déterminer la présence, voire le sexe, de tout autre embryon d'ovipare dont l'œil ou un autre organe présente une coloration par la mélanine.

La procédé et le système de l'invention sont basés sur une analyse par imagerie, et non par analyse de données spectrales.

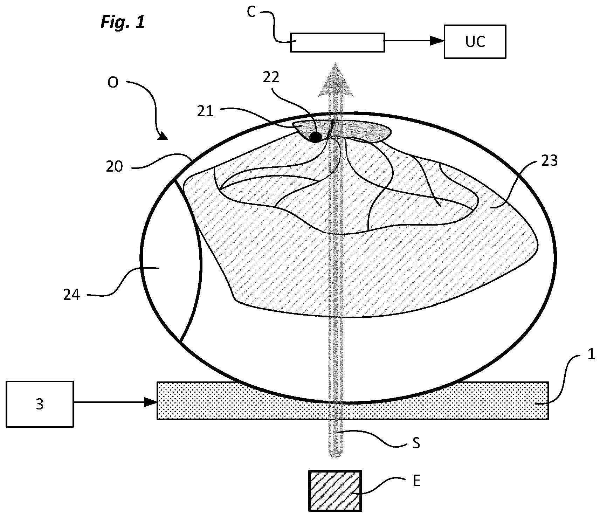

De manière connue, en référence à la figure 1, l'œuf O comporte une coquille 20, dans laquelle est présent l'embryon 21 auquel appartient l'œil 22, et le système cardio-vasculaire 23 contenant notamment de l'hémoglobine. L'oeuf comporte également une chambre à air 24 qui est située du côté opposé de la pointe de l'œuf. Sur la figure 1, l'œuf O est représenté à l'horizontale, la chambre à air 24 positionnée sur le côté.

En référence aux figures annexées, le système de l'invention, également montré sur la figure 1, se compose principalement de :
- Un élément de maintien configuré pour maintenir l'œuf O en position lors de la mise en oeuvre du procédé ;
- Une source d'émission E d'un signal lumineux S à destination de l'œuf ;
- Un dispositif d'imagerie comprenant un capteur d'images C positionné pour recevoir un flux lumineux après passage à travers l'œuf O ;
- Des moyens de traitement UC de données représentatives du flux lumineux reçu par le capteur d'images C ;

L'élément de maintien peut être un élément support 1 sur lequel l'œuf O est posé (comme illustré sur les figures annexées). Il pourrait aussi s'agir d'une pince ou de tout autre moyen équivalent.

Dans certaines configurations (voir ci-après), l'élément support 1 est choisi pour laisser passer le signal lumineux S émis par la source d'émission E, Il peut ainsi être choisi transparent pour laisser passer par exemple au moins 90% du signal lumineux, avec un minimum de diffusion, et/ou par exemple avec une ouverture par laquelle le signal lumineux S est émis, et/ou présenter au moins une partie en forme de grille destinée à être traversée par le signal. Ceci peut s'avérer nécessaire lorsque la source d'émission E est placée sous l'élément support 1 ou lorsque le capteur d'images C est placé sous l'élément support 1.

D'une manière générale, l'élément de maintien de l'œuf utilisé avoir le moins d'interaction possible avec le signal lumineux.

On veillera également à limiter l'extension spatiale de la zone de l'œuf éclairée par la source d'émission E afin d'éviter que des rayons lumineux ne soient diffusés par les bords de l'œuf ou par des éléments situés dans la proximité de l'œuf.

La solution de l'invention s'appuie sur la capacité d'absorption de la lumière visible par la mélanine présente à l'intérieur de l'œuf O pour créer un contraste suffisant sur l'image capturée par le capteur d'images C.

A titre d'exemple, l'œil d'un canard mulard de sexe mâle contient de la mélanine alors que celui de la femelle n'en contient pas.

Afin de faciliter la détection, le flux lumineux reçu par le capteur d'images C après son passage à travers l'œuf est limité à une plage de longueurs d'onde autour de 700nm. Pour cela, différentes options sont possibles :
- La source d'émission E peut être choisie pour émettre un signal lumineux S sur une plage de longueurs d'onde centrée sur 700nm.
- La source d'émission E peut être une source lumineuse éclairant sur une plage de longueurs d'onde étendue (par exemple visible ou visible et proche infrarouge), un filtre ou un ensemble de filtres étant placé entre la source et l'œuf pour ne conserver que la plage de longueurs d'ondes centrée autour de 700nm.
- La source d'émission peut être une source éclairant sur une plage de longueurs d'onde étendue, un filtre ou un ensemble de filtres étant placé entre l'œuf et le capteur pour filtrer le flux lumineux destiné à être reçu par le capteur pour ne conserver que la plage de longueurs d'onde centrée autour de 700nm.
- Une combinaison de ces différentes solutions, dès l'instant que le capteur reçoit un flux lumineux sur une plage de longueurs d'onde centrée autour de 700nm.

A titre d'exemple, une combinaison de deux filtres peut consister à prendre un filtre passe-bas qui laisse passer les longueurs d'ondes inférieures à 720nm et un filtre passe-haut qui laisse passer les ondes supérieures à 670nm.

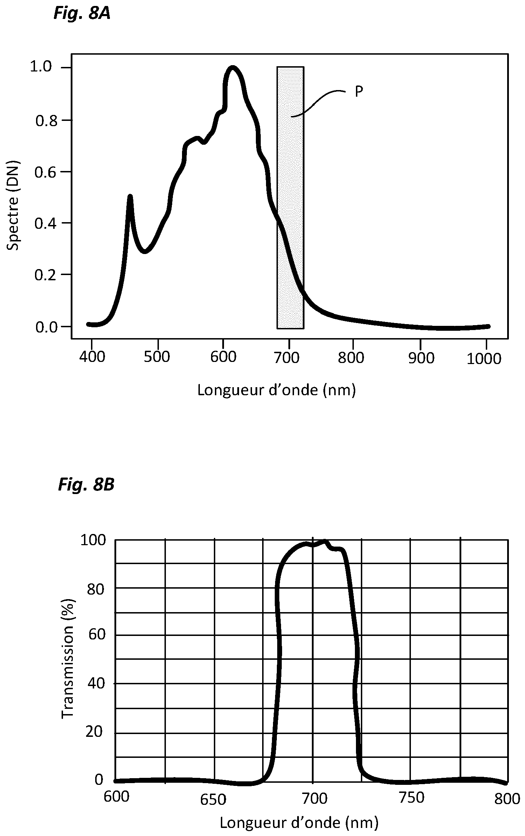

La source lumineuse peut comporter une ou plusieurs diodes électroluminescentes. De manière non limitative, selon la deuxième option décrite ci-dessus, la source lumineuse est par exemple choisie pour émettre avec un spectre tel que celui représenté par la courbe de la figure 8A.

Sur cette figure 8A, la plage d'intérêt P autour de 700nm est identifiée.

La figure 8B montre un exemple de réponse de filtre de type passe bande autour de la plage d'intérêt de 700nm, qui peut être appliqué sur la source lumineuse. Préférentiellement, on choisira un filtre passe-bande ou en ensemble de filtres passe-haut et passe-bas qui définissent une fenêtre de transmission de 50nm de largeur entre 675nm et 725nm afin d'optimiser le contraste entre la mélanine et l'oxyhémoglobine contenu dans l'embryon. Plus la fenêtre spectrale s'élargira plus le contraste diminuera mais plus le flux reçu par le capteur d'images C sera important. Avantageusement, on ne dépassera pas une largeur de fenêtre de 100nm entre 650nm et 750nm pour maintenir un contraste d'absorption d'au moins 10 entre la mélanine et l'oxyhémoglobine.

Le capteur C est avantageusement une caméra de type monochrome, de manière à pouvoir bien identifier le contraste entre les zones de l'œuf O où la lumière est transmise et les zones de l'œuf O où la lumière est absorbée.

En ce qui concerne le sexage d'un oeuf O de canard mulard, l'utilisation d'une plage P étroite de longueurs d'ondes, autour de 700nm, permet d'avoir un contraste d'absorption maximal entre la mélanine contenue dans les yeux des embryons mâles et les autres constituants biologiques de l'œuf, notamment avec l'hémoglobine du système cardio-vasculaire, cette dernière présentant un minimum d'absorption autour de 700nm. Par ce procédé, le système cardio-vasculaire 23 disparait quasiment de l'image obtenue au niveau du capteur, permettant une identification quasiment systématique de l'œil des embryons mâles et donc l'identification du sexe de l'œuf. Le même principe peut s'appliquer pour déterminer la présence d'embryons d'ovipares présentant un organe coloré par la mélanine. La détection de l'embryon peut permettre la détermination de l'état de fécondation des oeufs voire de les sexer dans le cas où la présence de mélanine est corrélée au sexe de l'embryon.

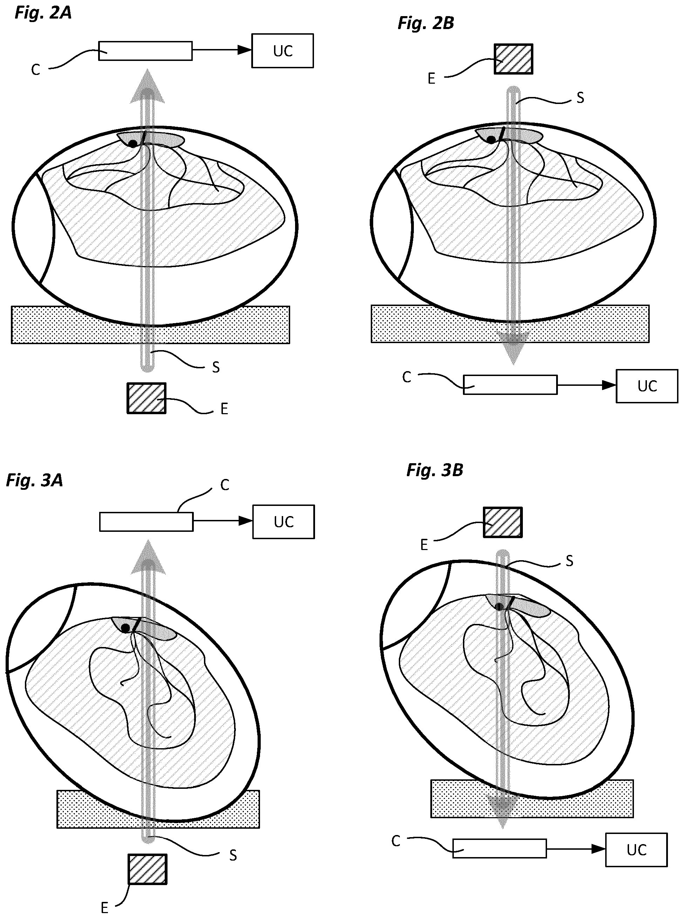

Dans une première configuration préférée illustrée par la figure 2A et la figure 2B, l'élément support 1 est configuré de sorte que l'œuf O est positionné à plat, c'est-à-dire avec son plus grand axe orienté à l'horizontal. Cette configuration permet d'optimiser l'éclairage de l'œuf O et d'avoir le meilleur positionnement de l'embryon par rapport au capteur d'images C, l'embryon 21 flottant généralement près du point le plus haut de l'œuf.

Dans cette première configuration, le flux lumineux est capté par le capteur (la caméra par exemple) qui est positionnée au-dessus (figure 2A) ou au-dessous (figure 2B), dans l'axe du signal émis par la source d'émission S. La source d'émission est ainsi respectivement placée au-dessous de l'élément support 1 (figure 2A) ou au-dessus de l'œuf (figure 2B). Le flux lumineux capté est ici très homogène car tous les rayons qui parviennent au capteur d'images C ont traversé une portion à peu près équivalente de l'œuf (à la géométrie de l'œuf près). Tous les rayons traversent également les mêmes composantes de l'œuf (blanc, jaune, coquille).

Dans une deuxième configuration représentée sur la figure 3A et sur la figure 3B, l'élément support 1 est adapté pour supporter l'œuf de sorte que son grand axe soit incliné à 45° par rapport à la verticale. Cette configuration correspond à l'inclinaison des oeufs pendant la phase d'incubation en couvoir, il n'est donc pas nécessaire d'attendre que l'embryon migre pour faire son image. Dans cette deuxième configuration, le flux lumineux est capté par le capteur (la caméra par exemple) qui peut être positionnée au-dessus (figure 3A) ou au-dessous (figure 3B), dans l'axe du signal émis par la source d'émission S située à l'opposé. Le flux lumineux capté est un peu moins homogène que dans la première configuration car les rayons parcourent des distances différentes entre le bas et le haut de l'œuf. Par ailleurs, l'embryon qui se retrouve plus proche de la chambre à air 24 peut être moins visible.

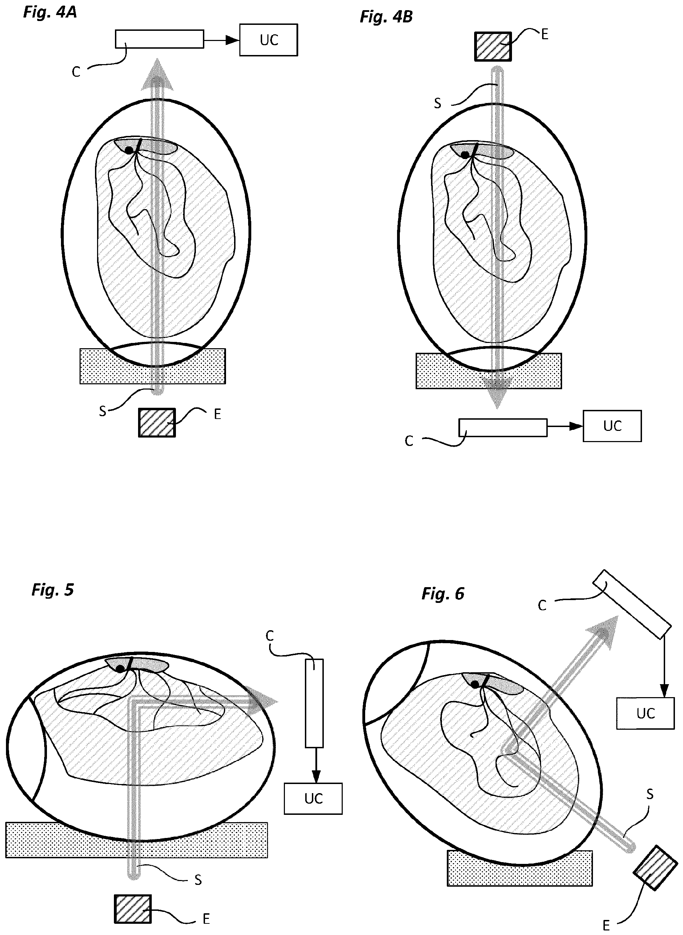

Dans une troisième configuration représentée sur la figure 4A et la figure 4B, l'élément support 1 est adapté pour maintenir l'œuf O de sorte que son plus grand axe soit orienté à la verticale et que la chambre à air 24 soit positionnée en bas.

Dans cette troisième configuration, le flux lumineux est capté par le capteur (la caméra par exemple) qui peut être positionnée au-dessus (figure 4A) ou au-dessous (figure 4B), dans l'axe du signal émis par la source d'émission S située à l'opposé. Cette troisième configuration permet d'avoir un éclairage assez homogène (comme dans la première configuration ci-dessus) mais le trajet du faisceau lumineux étant plus important, le flux collecté par le capteur d'images C est plus faible.

Dans ces trois premières configurations, l'élément support 1 est par exemple choisi transparent (ou autres solutions décrites ci-dessus).

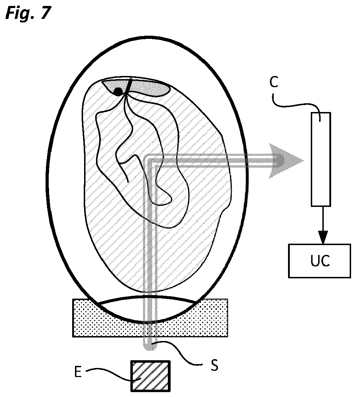

Les trois configurations décrites ci-dessus peuvent être déclinées en plusieurs autres configurations, dans lesquelles la source d'émission E et le capteur d'images C sont positionnés à 90° l'un par rapport à l'autre. La figure 5 montre ainsi ce type de configuration avec l'œuf positionné à l'horizontale et la source d'émission E émettant à la verticale, la figure 6 avec l'œuf orienté à 45° par rapport à la verticale et la source d'émission E émettant à 45° suivant le grand axe de l'œuf, et la figure 7 avec l'œuf à la verticale et la source d'émission E émettant à la verticale.

Bien entendu, il est également possible de décliner ces trois autres configurations des figures 5, 6 et 7 en inversant la position de la source d'émission E et du capteur d'images C, ainsi que en positionnant la source d'émission E et le capteur d'images C de manière symétrique.

Enfin, dans certaines configurations, il faut noter que la chambre à air 24 peut être située à l'opposé, c'est-à-dire vers le haut de l'œuf O, dans le cas où l'œuf est maintenu à la verticale.

De manière avantageuse, on veillera à plaquer la source au plus près de l'œuf pour éviter que des rayons ne soient réfléchis par la coquille et n'atteignent directement le capteur d'images C sans avoir traversé l'œuf O.

Bien entendu, d'autres configurations pourraient être envisagées, selon le type d'oeuf à caractériser, la caractéristique de l'œuf O à déterminer, les contraintes techniques de l'installation.

Selon une particularité, il est également possible de prévoir des moyens 3 pour mettre en mouvement de manière relative l'élément support 1, la source d'émission E et le capteur d'images C (moyens 3 illustrés sur la figure 1).

Ces moyens 3 de mise en mouvement peuvent être employés pour que l'embryon 21 vienne se placer au plus proche du capteur d'images C. De manière simple, il peut s'agir de moyens vibrants et/ou oscillants chargés de mettre en mouvement l'élément support 1 pour que l'embryon 21 se replace systématiquement vers le haut, par flottaison. Dans le cas où le capteur d'images C est situé dans l'axe de la source d'émission E (première configuration ci-dessus - figure 2A), avec la source d'émission E qui éclaire par le bas, on vient alors capter un maximum d'informations. Bien entendu, on comprend que d'autres options de mise en mouvement pourraient être envisagées, l'objectif étant d'optimiser la prise d'informations à l'aide du système de l'invention. Il serait notamment possible de déplacer le capteur d'images C par rapport à l'œuf O pour que celui-ci vienne se placer au plus près de l'embryon 21. Des moyens de déplacement du capteur d'images C pourraient ainsi être implémentés. Il serait également possible de prévoir une combinaison des différents moyens, c'est-à-dire des moyens 3 de mise en mouvement de l'élément support et des moyens de déplacement du capteur d'images C, afin de pouvoir s'adapter à toutes les conditions.

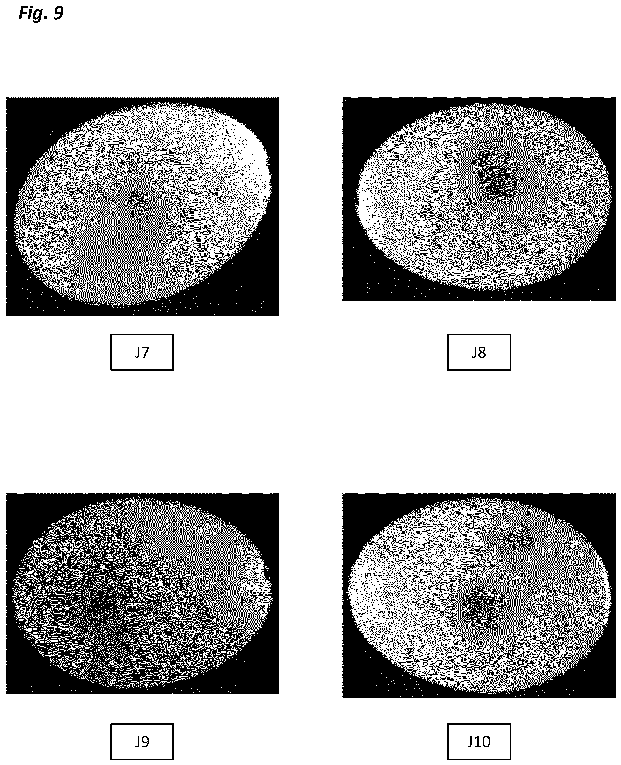

La figure 9 montre plusieurs photos prises par le capteur d'images C à différents stades d'incubation d'un oeuf (à J7, J8, J9, J10), dans le cas de la première configuration décrite ci-dessus. La zone sombre représente le composant biologique contenant la mélanine, absorbant le signal émis sur la plage de longueurs d'onde centrée sur 700nm. Dans le cas d'un canard mulard de sexe mâle, il s'agit de son oeil (tâche noire sur les images représentées). On constate ainsi que cet oeil reste toujours parfaitement détectable, dès le 7^{ème} jour d'incubation et jusqu'au 10^{ème} jour.

Ainsi, en choisissant de filtrer les longueurs d'onde autour de 700nm, on obtient un contraste très important entre la mélanine et l'hémoglobine (rapport de 50 dans les coefficients d'absorption), ce qui fait parfaitement ressortir l'œil.

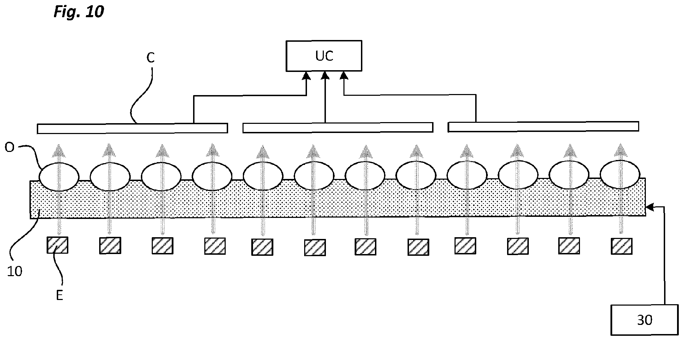

En référence à la figure 10, une installation complète permet de traiter plusieurs oeufs en même temps. Un plateau support 10 peut disposer de plusieurs emplacements distincts, destinés chacun au support d'un oeuf O distinct à caractériser. Une source d'émission E distincte peut être employée par emplacement et donc par oeuf O à caractériser. Un même capteur d'images C peut être commun à plusieurs oeufs à caractériser (par exemple pour 4 oeufs sur l'installation). Les moyens de traitement UC sont ensuite chargés de traiter les images qui ont été capturées par chaque capteur de l'installation, selon les principes définis ci-dessus.

Des moyens 30 de mise en mouvement du plateau support 10 peuvent être configurés, pour générer des accélérations, afin de repositionner l'embryon 21 de chaque oeuf O vers le haut et assurer ainsi qu'il soit au plus près de la coquille et donc du capteur d'images C lors de la lecture du flux lumineux transmis.

Les autres particularités du système déjà décrites ci-dessus sont applicables de manière identique à l'installation.

La solution de l'invention présente ainsi de nombreux avantages :
- Elle permet de caractériser un oeuf de manière fiable, non-invasive, à un stade précoce de l'incubation ;
- Elle utilise des moyens simples et fiables ;
- Elle est adaptable aux installations déjà existantes ;

## Revendications

1. Procédé non-invasif de détection de la présence d'un organe d'un embryon au sein d'un oeuf (O), comportant:
- Une étape d'émission à travers l'œuf, par une source d'émission (E), d'un signal lumineux (S), **caractérisé en ce qu'**il comporte encore:
- Une étape de filtrage du flux lumineux dans une bande spectrale étroite autour d'une longueur d'onde de 700nm,
- Une étape d'imagerie de l'œuf (O) traversé par le flux lumineux,
- Une étape d'analyse de l'image de l'œuf (O) générée lors de l'étape d'imagerie,
- La présence dudit organe étant révélée par contraste d'absorption entre les composantes constitutives de l'œuf (O) contenant du sang et ledit organe de l'embryon contenant de la mélanine.

2. Procédé selon la revendication 1, **caractérisé en ce que** la bande spectrale étroite est comprise entre 650nm et 750nm.

3. Procédé selon la revendication 1, **caractérisé en ce que** la bande spectrale étroite est comprise entre 675nm et 725nm.

4. Procédé selon l'une des revendications 1 à 3, **caractérisé en ce que** l'étape de filtrage est mise en oeuvre sur le flux lumineux émis par la source d'émission (E), avant transmission dans l'œuf.

5. Procédé selon l'une des revendications 1 à 3, **caractérisé en ce que** l'étape de filtrage est mise en oeuvre sur le flux lumineux émis par la source d'émission (E), après transmission à travers l'œuf et avant l'étape d'imagerie.

6. Procédé selon la revendication 1, **caractérisé en ce qu'**il comporte une étape mise en mouvement de l'œuf, contenant des accélérations de l'œuf (O) devant la source d'émission afin de ramener ledit organe contenant de la mélanine au plus près de la coquille.

7. Procédé selon l'une des revendications 1 à 6, **caractérisé en ce qu'**il consiste à déterminer le sexe d'un oeuf de canard mulard à partir de la détection de présence de l'œil dudit canard.

8. Système non-invasif de détection de la présence d'un organe d'un embryon au sein d'un oeuf (O), comportant:
- Un élément de maintien agencé pour maintenir l'œuf dans une position,
- Une source d'émission d'un signal lumineux (S) à travers l'œuf maintenu en position, **caractérisé en ce qu'**il comporte encore:
- Un dispositif de réduction de la bande spectrale du signal lumineux émis par la source dans une bande spectrale étroite autour d'une longueur d'onde de 700nm,
- Un dispositif d'imagerie configuré et agencé pour recevoir un flux lumineux après transmission à travers l'œuf,
- Des moyens d'analyse de l'image de l'œuf (O) obtenue à l'aide du dispositif d'imagerie, configurés pour détecter au sein de l'œuf un contraste d'absorption entre les composantes constitutives de l'œuf (O) contenant du sang et ledit organe de l'embryon contenant de la mélanine.

9. Système selon la revendication 8, **caractérisé en ce que** le dispositif de réduction de la bande spectrale est un filtre centré sur une longueur d'ondes de 700nm et de largeur de bande de 100nm.

10. Système selon l'une des revendications 8 à 10, **caractérisé en ce que** le dispositif de réduction de la bande spectrale est situé entre la source d'émission (E) et l'élément de maintien en position de l'œuf.

11. Système selon l'une des revendications 8 à 10, **caractérisé en ce que** le dispositif de réduction de la bande spectrale est situé entre l'élément de maintien en position de l'œuf et le dispositif d'imagerie.

12. Système selon l'une des revendications 8 à 11, **caractérisé en ce qu'**il comporte des moyens de mise en mouvement de l'élément de maintien, configurés pour créer des accélérations.

13. Système selon l'une des revendications 8 à 12, **caractérisé en ce que** l'élément de maintien est configuré pour maintenir l'oeuf de sorte que le plus grand axe de l'œuf soit parallèle à un plan horizontal.

14. Système selon la revendication 13, **caractérisé en ce que** la source d'émission (E) est positionnée pour émettre le signal lumineux (S) suivant l'axe d'émission perpendiculaire audit plan horizontal.

15. Système selon la revendication 14, **caractérisé en ce que** la source d'émission (E) est positionnée de manière à pouvoir éclairer l'œuf (O) par le bas.

16. Système selon l'une des revendications 13 à 15, **caractérisé en ce que** le capteur (C) est positionné dans l'axe d'émission de la source d'émission (E).

17. Système selon l'une des revendications 13 à 15, **caractérisé en ce que** le capteur (C) est positionné pour recevoir le flux lumineux à 90° par rapport à l'axe d'émission.

18. Utilisation du système tel que défini dans l'une des revendications 8 à 17, pour déterminer le sexe d'un oeuf de canard.

## Patentansprüche

1. Nicht-invasives Verfahren zur Erkennung der Anwesenheit eines Organs eines Embryos in einem Ei (O), umfassend:
- einen Schritt des Emittierens eines Lichtsignals (S) aus einer Emissionsquelle (E) durch das Ei, **dadurch gekennzeichnet, dass** es ferner umfasst:
- einen Schritt der Filterung des Lichtflusses in ein schmales Spektralband rund um eine Wellenlänge von 700 nm,
- einen Schritt der Bildgebung des vom Lichtfluss durchstrahlten Eies (O),
- einen Schritt des Analysierens des Bilds des Eies (O), das in dem Bildgebungsschritt erstellt wurde,
- wobei die Anwesenheit des Organs durch den Absorptionskontrast zwischen den Blut enthaltenden Bestandteilen des Eies (O) und dem Melanin enthaltenden Organ des Embryos aufgezeigt wird.

2. Verfahren nach Anspruch 1, **dadurch gekennzeichnet, dass** das schmale Spektralband von 650 nm bis 750 nm reicht.

3. Verfahren nach Anspruch 1, **dadurch gekennzeichnet, dass** das schmale Spektralband von 675 nm bis 725 nm reicht.

4. Verfahren nach einem der Ansprüche 1 bis 3, **dadurch gekennzeichnet, dass** der Schritt der Filterung an dem aus der Emissionsquelle (E) emittierten Lichtfluss vor der Transmission in das Ei durchgeführt wird.

5. Verfahren nach einem der Ansprüche 1 bis 3, **dadurch gekennzeichnet, dass** der Schritt der Filterung an dem aus der Emissionsquelle (E) emittierten Lichtfluss nach der Transmission durch das Ei und vor dem Bildgebungsschritt durchgeführt wird.

6. Verfahren nach Anspruch 1, **dadurch gekennzeichnet, dass** es einen Schritt der Bewegung des Eies, einschließlich Beschleunigungen des Eies (O) vor der Emissionsquelle, umfasst, um das Melanin enthaltende Organ in nächste Nähe zur Schale zu bringen.

7. Verfahren nach einem der Ansprüche 1 bis 6, **dadurch gekennzeichnet, dass** es aus der Geschlechtsbestimmung eines Mulardenten-Eies ab Erkennung der Anwesenheit des Eies der Ente besteht.

8. Nicht-invasives System zur Erkennung der Anwesenheit eines Organs eines Embryos in einem Ei (O), umfassend:
- ein Halteelement, angeordnet, um das Ei in einer Position zu halten,
- eine Quelle zur Emission eines Lichtsignals (S) durch das in Position gehaltene Ei, **dadurch gekennzeichnet, dass** es ferner umfasst:
- eine Vorrichtung zur Reduktion des Spektralbandes des aus der Quelle emittierten Lichtsignals in ein schmales Spektralband rund um eine Wellenlänge von 700 nm,
- eine Bildgebungsvorrichtung, die so konfiguriert und angeordnet ist, dass sie einen Lichtfluss nach der Transmission durch das Ei empfängt,
- Einrichtungen zum Analysieren des mithilfe der Bildgebungsvorrichtung erhaltenen Bilds des Eies (O), die dazu konfiguriert sind, innerhalb des Eies einen Absorptionskontrast zwischen den Blut enthaltenden Bestandteilen des Eies (O) und dem Melanin enthaltenden Organ des Embryos zu erkennen.

9. System nach Anspruch 8, **dadurch gekennzeichnet, dass** die Vorrichtung zur Reduktion des Spektralbandes ein Filter ist, der auf eine Wellenlänge von 700 nm und eine Bandbreite von 100 nm zentriert ist.

10. System nach einem der Ansprüche 8 bis 10, **dadurch gekennzeichnet, dass** die Vorrichtung zur Reduktion des Spektralbandes zwischen der Emissionsquelle (E) und dem Positions-Halteelement für das Ei platziert ist.

11. System nach einem der Ansprüche 8 bis 10, **dadurch gekennzeichnet, dass** die Vorrichtung zur Reduktion des Spektralbandes zwischen dem Positions-Halteelement für das Ei und der Bildgebungsvorrichtung platziert ist.

12. System nach einem der Ansprüche 8 bis 11, **dadurch gekennzeichnet, dass** es Einrichtungen zur Bewegung des Halteelements umfasst, die zum Erzeugen von Beschleunigungen konfiguriert sind.

13. System nach einem der Ansprüche 8 bis 12, **dadurch gekennzeichnet, dass** das Halteelement konfiguriert ist, um das Ei so zu halten, dass die längste Achse des Eies parallel zu einer horizontalen Ebene ist.

14. System nach Anspruch 13, **dadurch gekennzeichnet, dass** die Emissionsquelle (E) positioniert ist, um das Lichtsignal (S) entlang einer Emissionsachse senkrecht zur horizontalen Ebene zu emittieren.

15. System nach Anspruch 14, **dadurch gekennzeichnet, dass** die Emissionsquelle (E) so positioniert ist, dass das Ei (O) von unten beleuchtet werden kann.

16. System nach einem der Ansprüche 13 bis 15, **dadurch gekennzeichnet, dass** der Sensor (C) in der Emissionsachse der Emissionsquelle (E) positioniert ist.

17. System nach einem der Ansprüche 13 bis 15, **dadurch gekennzeichnet, dass** der Sensor (C) positioniert ist, um den Lichtfluss bei 90° relativ zur Emissionsachse zu empfangen.

18. Verwendung des Systems nach einem der Ansprüche 8 bis 17 zur Geschlechtsbestimmung eines Enteneies.

## Claims

1. Non-invasive method for detecting the presence of an organ of an embryo within an egg (O), comprising:
- a step of transmitting through the egg, by means of an emission source (E), a light signal (S), **characterized in that** it further comprises:
- a step of filtering the luminous flux in a narrow spectral band around a wavelength of 700 nm,
- a step of imaging of the egg (O) passed through by the luminous flux,
- a step of analysing the image of the egg (O) generated in the imaging step,
- the presence of said organ being revealed by absorption contrast between the blood-containing constituent components of the egg (O) and said melanin-containing organ of the embryo.

2. Method according to Claim 1, **characterized in that** the narrow spectral band is between 650 nm and 750 nm.

3. Method according to Claim 1, **characterized in that** the narrow spectral band is between 675 nm and 725 nm.

4. Method according to one of Claims 1 to 3, **characterized in that** the filtering step is carried out on the luminous flux emitted by the emission source (E), before transmission through the egg.

5. Method according to one of Claims 1 to 3, **characterized in that** the filtering step is carried out on the luminous flux emitted by the emission source (E), after transmission through the egg and before the imaging step.

6. Method according to Claim 1, **characterized in that** it comprises a step of moving the egg, containing accelerations of the egg (O) in front of the emission source in order to bring said melanin-containing organ as close as possible to the shell.

7. Method according to one of Claims 1 to 6, **characterized in that** it consists in determining the sex of a mulard duck egg based on detection of the presence of the eye of said duck.

8. Non-invasive system for detecting the presence of an organ of an embryo within an egg (O), comprising:
- a holding element arranged to hold the egg in a position,
- an emission source for transmitting a light signal (S) through the egg held in position, **characterized in that** it further comprises:
- a device for reducing the spectral band of the light signal emitted by the source to a narrow spectral band around a wavelength of 700 nm,
- an imaging device configured and arranged to receive a luminous flux after transmission through the egg,
- means for analysing the image of the egg (O) obtained using the imaging device, said means being configured to detect, within the egg, an absorption contrast between the blood-containing constituent components of the egg (O) and said melanin-containing organ of the embryo.

9. System according to Claim 8, **characterized in that** the device for reducing the spectral band is a filter centred on a wavelength of 700 nm and of a bandwidth of 100 nm.

10. System according to one of Claims 8 to 10, **characterized in that** the device for reducing the spectral band is located between the emission source (E) and the element for holding the egg in position.

11. System according to one of Claims 8 to 10, **characterized in that** the device for reducing the spectral band is located between the element for holding the egg in position and the imaging device.

12. System according to one of Claims 8 to 11, **characterized in that** it comprises means for setting the holding element in motion, which means are configured to create accelerations.

13. System according to one of Claims 8 to 12, **characterized in that** the holding element is configured to hold the egg so that the longest axis of the egg is parallel to a horizontal plane.

14. System according to Claim 13, **characterized in that** the emission source (E) is positioned to emit the light signal (S) along the emission axis perpendicular to said horizontal plane.

15. System according to Claim 14, **characterized in that** the emission source (E) is positioned so as to be able to illuminate the egg (O) from below.

16. System according to one of Claims 13 to 15, **characterized in that** the sensor (C) is positioned on the emission axis of the emission source (E).

17. System according to one of Claims 13 to 15, **characterized in that** the sensor (C) is positioned to receive the luminous flux at 90° with respect to the emission axis.

18. Use of the system such as defined in one of Claims 8 to 17, to determine the sex of a duck egg.
